# EUROPEAN PATENT APPLICATION

(11) **EP 4 840 895 A1**
(43) Date of publication of application: **18.11.2026**
(21) Application number: 24916238.9
(22) Date of filing: 11.04.2024
(51) Int. Cl.: C08G 65/337

(54) **PEGYLATED BORON-CONTAINING TRYPTOPHAN DERIVATIVE AND PREPARATION METHOD THEREFOR, PHARMACEUTICAL COMPOSITION CONTAINING PEGYLATED BORON-CONTAINING TRYPTOPHAN DERIVATIVE, AND USE THEREOF**

(30) Priority: 11.01.2024 US 202463619755 P
(71) Applicant: National Tsing Hua University, Hsinchu (TW)
(72) Inventor: Yu, Chung-Shan, Hsinchu City 300 (TW)
(74) Representative: Scholz, Volker
(86) International application number: PCT/CN2024/087223
(87) International publication number: WO 2025/148180

(57) **Abstract**

The present disclosure provides a PEGylated boronotryptophan derivative and the preparation process thereof, a pharmaceutical composition including the PEGylated boronotryptophan derivative, and uses of the PEGylated boronotryptophan derivative for treating tumor and for PET scanning for assessing pharmacokinetics for boron neutron capture therapy of tumors and quantifying drug concentrations to calculate pharmacokinetics. The PEGylated boronotryptophan derivative and the pharmaceutical composition including the PEGylated boronotryptophan derivative of the present disclosure achieve the effect of treating tumor and being used for PET scanning for assessing the pharmacokinetics for boron neutron capture therapy of tumors and quantifying drug concentrations to calculate pharmacokinetics through various efficacy experiments.

## Description

### FIELD OF THE INVENTION

The present invention relates to a PEGylated boronotryptophan derivative and the preparation process thereof, a pharmaceutical composition comprising the PEGylated boronotryptophan derivative, and uses thereof.

### THE PRIOR ARTS

As being the most malignant primary central nervous system tumor, the prognosis for Glioblastoma (GBM) patients is often bleak. In US, an estimated 40,594 children and adolescents ages 0-19 years were diagnosed as a primary brain and other central nervous system (CNS) tumor in 2022, almost comparable to the cases of leukemia as diagnosed in 40,738 children and adolescents ages 0-19 years. Furthermore, these diseases are incurable as evidenced by 2,693 deaths attributed to malignant brain and other CNS tumors between 2014 and 2018 in children and adolescents ages 0-19 years. This corresponds to an annual average mortality rate of 0.66 per 100,000 population, equivalent to average of 539 deaths per year caused by malignant brain and other CNS tumors.

GBM is currently treated using surgery, radiation therapy and chemotherapy of Temozolomide (TMZ) as the standard therapies. The median overall survival rate is only 14.6 months, however. In general, the overall 5-year survival rate is merely 6.8%. The very low survival rate may be caused by the infiltrative growth which cannot be completely resected. Other causes include the drug resistance or radiation insensitivity.

Contradicting to the drugs targeting the sites other than the brain, CNS drugs need to cross the blood brain barrier (BBB). The BBB is responsible for nutrient transport, homeostasis, and communication between the body and the brain and also prevents foreign substances from reaching the brain. Two types of cellular junctions such as intercellular adherens junctions and paracellular tight junctions halt passive diffusion and prevent leakiness between the endothelial cells.

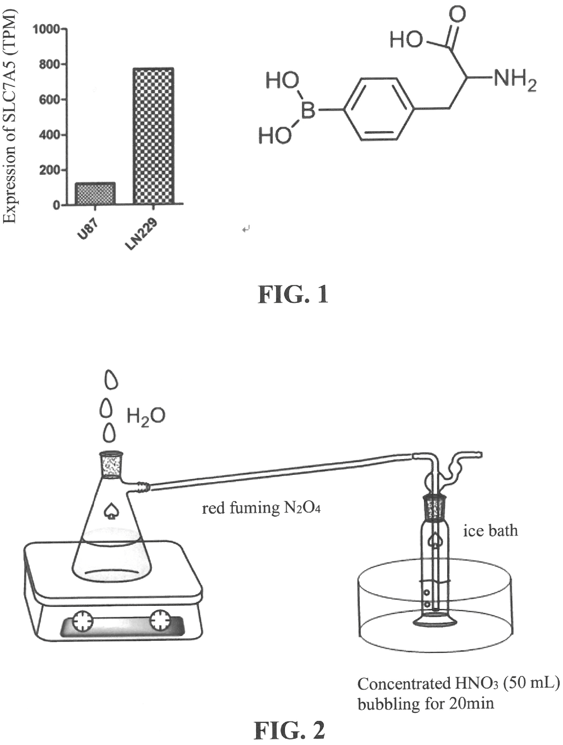

Nutrients and small molecules may be transported in and out of the brain by various methods, including passive diffusion, carrier-mediated transport, endocytosis, and active transport. Carrier-mediated transport is driven by two major protein families, the solute carrier (SLC) superfamily and ATP binding cassette (ABC) transporters. The main function of these transporters is to carry essential amino acids and glucose from the blood to the brain. SLC7A5 is a transporter dedicated to essential amino acids. In the pre-genomic era, it was known as LAT1, an acronym standing for Large Amino Acid Transporter 1. LAT-1 is able to mediate antiport of amino acids. Furthermore, LAT-1 was found to overexpress in brain tumors. Phenylalanine and tryptophan both as the essential amino acid nutrients for brain metabolism are thought to be vehicles for delivering boron-10 atoms for targeting boron neutron capture therapy (BNCT). Among which, boronophenyl alanine (BPA) has been studying intensively in preclinical stage (see FIG. 1).

Hence, tryptophan as an essential amino acid might be able to cross the BBB through LAT-1 transporter with respect to the nutrient interplay. The increasing lipophilicity through protecting groups can improve the penetration across BBB but also display undesired nonspecific accumulation in other organs such as liver. On the other hand, the decreasing hydrophilicity makes it an inappropriate formulation through tail vein injection as indicated in our previous work. Wherein, the tryptophan acts as a platform to deliver boron-10 to tumor site to perform BNCT. BNCT has been studied intensively for treatment of recurrent brain tumor. Enriched ¹⁰B atom covalently bonded to molecules with biological function can be accumulated in the tumor loci followed by bombardment using the epithermal neutrons with a penetrating range of 10 cm. This results in splitting of two high energetic particles of ⁷Li and alpha ion that can damage the surrounding tumor cells within a cell-diameter range.

To improve the above limited hydrophilicity of these tri-Boc 5- and 6-boronopinacol tryptophan compounds, a prodrug concept by conjugating polyethylene glycol (PEG) is reported herein. PEG is an FDA approved polymer that enhances surface hydrophilicity and reduces association and uptake by phagocytes. Comparing with the roles played by PEG in nanomedicine as mainly constructed through the coagulation with the bioactive agent or pharmaceuticals, covalently bonded PEG-drug molecules have been reported recently.

While up to date, there are some ways for indirect quantification of cellular uptake of PEGylated nanoparticles, like hybridization of metal nanoparticles and tags of fluorescent dyes, quantification of PEGylated small molecules in animals is relatively limited in scope. The available labeling or tagging PEGylated nanoparticles with dyes, isotopes, or metal NPs, often leads to significant changes in the physical-chemical properties, like size, stiffness, and surface groups. Without the disadvantages brought about by these qualitative tools, radioactive transition metals such as ⁶⁷Ga is thus suitable for assessing high molecular-weight biological molecules e.g. proteins or peptides through coordinative chelation. The geometric requirement for chelation, however, limits the biological recognition of small molecules.

In order to solve the above-mentioned problems, those skilled in the art urgently need to develop a novel PEGylated boronotryptophan derivative, a pharmaceutical composition comprising the PEGylated boronotryptophan derivative, and a method for assessing pharmacokinetics for boron neutron capture therapy of tumors (e.g., brain tumors) and quantifying drug concentrations to calculate pharmacokinetics for the benefit of a large group of people in need thereof.

### SUMMARY OF THE INVENTION

A primary objective of the present invention is to provide a PEGylated boronotryptophan derivative, having the following chemical formula (I): 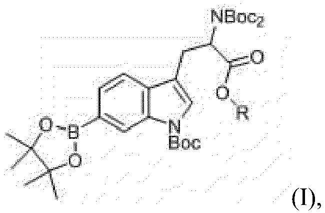 wherein R is polyethylene glycol (PEG).

According to an embodiment of the present invention, wherein the PEG is PEG₂₀₀, and when the PEG is PEG₂₀₀, the PEGylated borotryptophan derivative has the following chemical formula (Ia):

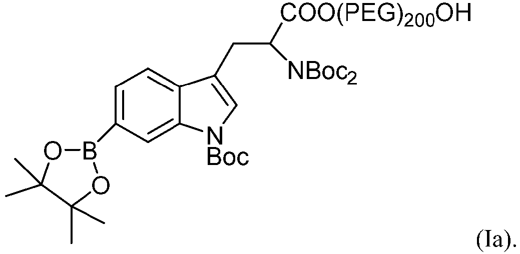

According to an embodiment of the present invention, the PEG is PEG₆₀₀, and when the PEG is PEG₆₀₀, the PEGylated borotryptophan derivative has the following chemical formula (Ib):

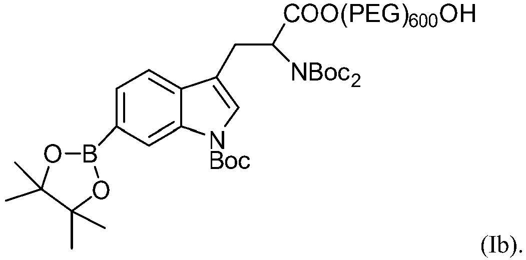

According to an embodiment of the present invention, the PEG is PEG₁₀₀₀, and when the PEG is PEG₁₀₀₀, the PEGylated borotryptophan derivative has the following chemical formula (Ic):

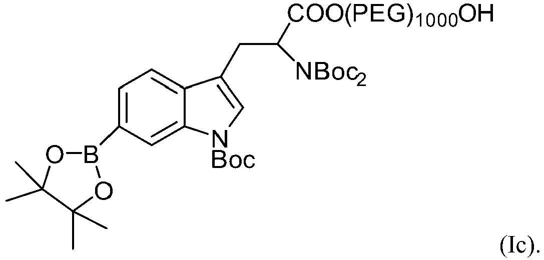

Another objective of the present invention is to provide a PEGylated boronotryptophan derivative, having the following chemical formula (II): 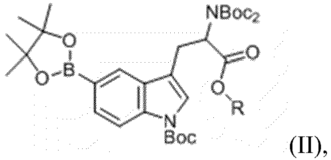 wherein R is polyethylene glycol (PEG).

According to an embodiment of the present invention, the PEG is PEG₂₀₀, and when the PEG is PEG₂₀₀, the PEGylated borotryptophan derivative has the following chemical formula (IIa):

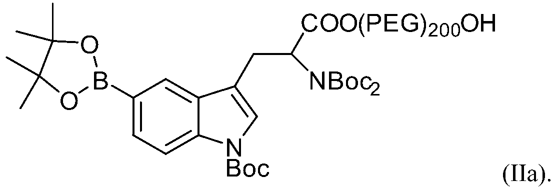

According to an embodiment of the present invention, the PEG is PEG₆₀₀, and when the PEG is PEG₆₀₀, the PEGylated borotryptophan derivative has the following chemical formula (IIb):

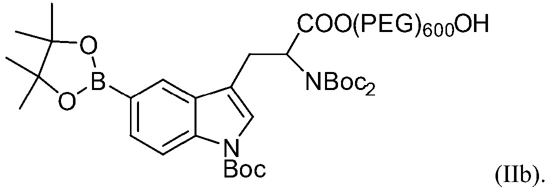

Another objective of the present invention is to provide a PEGylated boronotryptophan derivative, having the following chemical formula (III): 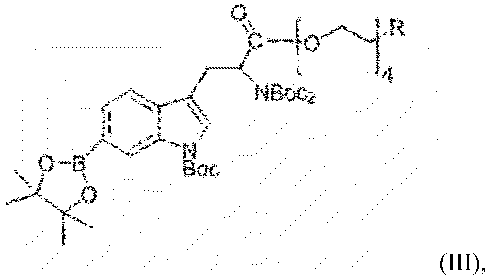 wherein R is OTs or F, and the PEGylated boronotryptophan derivative comprises PEG₂₀₀.

According to an embodiment of the present invention, when R is OTs, the PEGylated boronotryptophan derivative has the following chemical formula (IIIa):

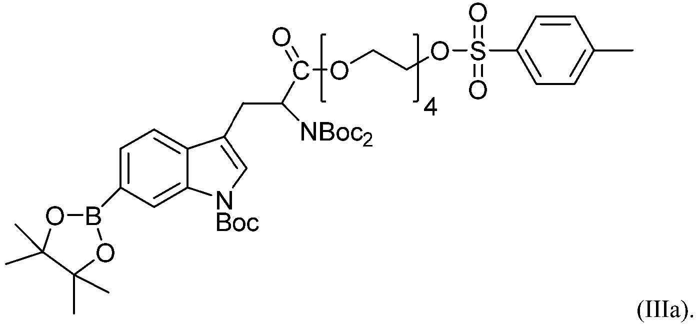

According to an embodiment of the present invention, when R is F, the PEGylated boronotryptophan derivative has the following chemical formula (IIIb):

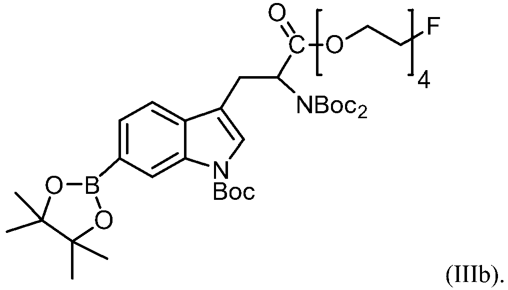

According to an embodiment of the present invention, the F is radioactive isotope of fluorine-18 (¹⁸F).

Another objective of the present invention is to provide a method for preparing the above mentioned PEGylated boronotryptophan derivative, comprising adding HBTU, DMAP and NEt₃ to the compound of chemical formula (I), wherein R of the compound of chemical formula (I) is H.

Another objective of the present invention is to provide a method for preparing the above mentioned PEGylated boronotryptophan derivative, comprising adding HBTU, DMAP and NEt₃ to the compound of chemical formula (II), wherein R of the compound of chemical formula (II) is H.

Another objective of the present invention is to provide a pharmaceutical composition, comprising the above mentioned PEGylated boronotryptophan derivative, and a pharmaceutically acceptable carrier.

According to an embodiment of the present invention, the pharmaceutical composition is in a dosage form for parenteral administration.

Another objective of the present invention is to provide a use of the above mentioned PEGylated boronotryptophan derivative for the manufacture of a medicament for treating tumor.

Another objective of the present invention is to provide a use of the above mentioned PEGylated boronotryptophan derivative for the manufacture of a formulation for positron emission tomography (PET) scanning for assessing pharmacokinetics for boron neutron capture therapy (BNCT) of tumor and quantifying drug concentrations to calculate pharmacokinetics.

According to an embodiment of the present invention, the tumor is a brain tumor.

According to an embodiment of the present invention, the brain tumor is glioma.

Another objective of the present invention is to provide a method for assessing pharmacokinetics for boron neutron capture therapy of tumor, comprising using the above mentioned PEGylated boronotryptophan derivative for positron emission tomography (PET) scanning.

In summary, the effects of the PEGylated boronotryptophan derivative and the pharmaceutical composition comprising the PEGylated boronotryptophan derivative of the present invention are as follows: The results illustrated in the following examples are suitable for the development of diagnostic and therapeutic molecules with high drug accumulation in living animal tumor models, wherein the high tumor cumulative dose, that is, 18% of the drug is accumulated in the tumor, and the high tumor to normal tissue accumulation ratio is 100:1; the toxicity is extremely low, that is, the cell survival rate is still more than 90% at 1000 µM, thereby achieving the effects on treating tumor (e.g., brain tumor) and for positron emission tomography (PET) scanning for assessing pharmacokinetics for boron neutron capture therapy (BNCT) of tumors and quantifying drug concentrations to calculate pharmacokinetics.

The embodiments of the present invention will be further described below. The following examples are used to illustrate the present invention and are not intended to limit the scope of the present invention. Anyone skilled in the art can make some changes and modifications without departing from the spirit and scope of the present invention. Therefore, the scope of the present invention shall be defined by the appended claims.

### BRIEF DESCRIPTION OF DRAWINGS

FIG. 1 shows that higher Large Amino Acid Transporter 1 (LAT-1) expression level in both glioma cell lines U87 and LN229 as depicted according to the database of genome and structure of boronophenyl alanine (BPA) used for boron neutron capture therapy (BNCT) is shown.
FIG. 2 shows the preparation process of the N₂O₄ saturated nitric acid (red fuming HNO₃) required for the nitration step.
FIG. 3 shows the scheme 1 of boronotryptophan compound 1 (TB-6-BTme 1), in which (a) LiOH, THF/H₂O 2:1, 0 °C, rt for 4-16 h, 73-93% yield (TB-6-BT **3** and TB-5-BT **4**), (b) HBTU (4 eq), DMAP (4 eq), NEt₃ (100 eq), PEG200-1000 (2 eq), 20 h, 30-60% yield (TB-6-BT-PEG₂₀₀ **5**-200, TB-6-BT-PEG₆₀₀ **5**-600, TB-6-BT-PEG₁₀₀₀ **5**-1000, TB-5-BT-PEG₂₀₀ **6**-200, and TB-5-BT-PEG₆₀₀ **6**-600).
FIG. 4 shows the scheme 2 of preparation of the tosylated pegylated TB-6-BT **7**, followed by fluorination and radiofluorination. (a) TsCl, toluene, rt for 3h, 61% yield (TB-6-BT-PEG₂₀₀OTs **7**). (b) KF, DMF, 100 °C for 20 min. (c) H[¹⁸F]F, K₂CO₃, Kryptofix[2,2,2], CH₃CN, 1.7% corrected radiochemical yield (EOB) (radioactive compound **[¹⁸F]F-8**).
FIG. 5 shows the cell survival assay using different boronotryptophan analogs and controls. Three different concentrations (one concentration for each compound, determined by the compound's water solubility) were specified as (a), (b) and (c) due to different aqueous solubility. (a) 500 µM+0.8% DMSO, (b) 1000 µM+0.8% DMSO and (c) 2000 µM+0.8% DMSO.
FIGs. 6A and 6B show cellular accumulation of the boron-containing compounds by using three different concentrations (one concentration for each compound, determined by the compound's water solubility) and specifying as (a) 100 µM + 0.8% DMSO, (b) 250 µM + 0.8% DMSO, and (c) 482 µM + 0% DMSO. The two human glioblastoma cell lines U87 (FIG. 6A) and LN229 (FIG. 6B) were incubated for 1 h, 2 h, and 4 h, respectively.
FIG. 7 shows a series of PET imaging taken for temporal distribution of TB-6-BTPEG₂₀₀[¹⁸F]F (**[¹⁸F]F-8)** in the mouse of xenograft model with a tumor cell line of U87.
FIG. 8A shows PET image-derived radioactivity distribution of TB-6-BTPEG₂₀₀[¹⁸F]F (**[¹⁸F]F-8**) in the mouse of xenograft model with a tumor cell line of U87. FIG. 8B shows radioactivity uptake data from the tumor and muscle sites to generate the temporal uptake ratio.
FIG. 9 shows a series of PET imagings taken for temporal distribution of TB-6-BTPEG₂₀₀[¹⁸F]F (**[¹⁸F]F-8**) in the mouse of xenograft model with a tumor cell line of LN229.
FIG. 10A shows PET image-derived radioactivity distribution of TB-6-BTPEG₂₀₀[¹⁸F]F (**[¹⁸F]F-8**) in the mouse of xenograft model with a tumor cell line of LN229. FIG. 10B shows radioactivity uptake data from the tumor and muscle sites to generate the temporal uptake ratio.
FIG. 11 shows PET imaging for TB-6-BTPEG₂₀₀[¹⁸F]F merged from 0 to 60 min in the mouse of xenograft model with a tumor cell line of U87 and LN229.
FIG. 12 shows size distribution of boronotryptophan derivatives measured by the laser scattering particle size analyzer.
FIG. 13 shows comparison of the aqueous solubility of the pegylated tryptophan analogs.
FIG. 14 shows proposed coagulation of the pegylated boronotryptophan analogs.

### DETAILED DESCRIPTION OF THE PREFERRED EMBODIMENT

### Definition

As used herein, the data provided represent experimental values that can vary within a range of ±10%, preferably within ±5%.

Unless otherwise stated in the context, "a", "the" and similar terms used in the specification (especially in the following claims) should be understood as including singular and plural forms.

As used herein, the term "treating" or "treatment" refers to alleviating, reducing, ameliorating, relieving or controlling one or more clinical signs of a disease or disorder, and lowering, stopping, or reversing the progression of severity regarding the condition or symptom being treated.

According to the present invention, the pharmaceutical composition can be manufactured to a dosage form suitable for parenteral administration, using techniques well known to those skilled in the art, including, but not limited to, injection (e.g., sterile aqueous solution or dispersion), sterile powder, tablet, troche, lozenge, pill, capsule, dispersible powder or granule, solution, suspension, emulsion, syrup, elixir, slurry, and the like.

The pharmaceutical composition according to the present invention may be administered by a parenteral route selected from the group consisting of: intraperitoneal injection, subcutaneous injection, intraepidermal injection, intradermal injection, intramuscular injection, intravenous injection, and intralesional injection.

According to the present invention, the pharmaceutical composition may further comprise a pharmaceutically acceptable carrier which is widely used in pharmaceutically manufacturing techniques. For example, the pharmaceutically acceptable carrier can comprise one or more reagents selected from the group consisting of solvent, emulsifier, suspending agent, decomposer, binding agent, excipient, stabilizing agent, chelating agent, diluent, gelling agent, preservative, lubricant, absorption delaying agent, liposome, and the like. The selection and quantity of these reagents fall within the scope of the professional literacy and routine techniques of those skilled in the art.

According to the present invention, the pharmaceutically acceptable carrier comprises a solvent selected from the group consisting of water, normal saline, phosphate buffered saline (PBS), sugar-containing solution, aqueous solution containing alcohol, and combinations thereof.

According to the present invention, as being the member of the 2^{nd} periodic atom, radiofluorine e.g. ¹⁸F atom (t_{1/2} = 110 min) could be covalently bonded to the small molecules or drugs without hampering the molecular shape and thus still preserving the biological recognition. In addition, like Ga-67, F-18 is a positron emitter that can capture surrounding electrons to emit two 180° angled coherent gamma radiations thereby enabling internal calibration for reconstructing the 3-D images and the pharmacokinetics. Thus, the ¹⁰B concentration of small molecular boron compounds in tumor loci can be derived and a treatment plan can be made to optimize the neutron dosage before BNCT. We are therefore interested in introducing ¹⁸F atom into the 6-borono tryptophan. Due to the labile boronopinacol group, electrophilic fluorination using [¹⁸F]F₂/Ne failed to give any trace of fluorinated product but only decomposition. The appropriate site for nucleophilic fluorination is only realizable when introducing PEG group. This may not only provide the terminal hydroxy group available for tosyl functionalization but also improve its aqueous solubility.

The present invention is further illustrated by the following examples. These examples are provided for illustration only and are not intended to limit the scope of the present invention. The scope of the present invention is shown in the appended claims.

The general experimental process is as follows. All reagents and solvents were purchased from Acros (Geal, Belgium) and Alfa (Binfield, Berkshire, UK). PEG 200 and 600 were purchased from First Chemicals Ltd, local company of Taiwan (China). PEG 1000 was purchased from Acros (Geal, Belgium). Preparation of all compounds was routinely conducted in dried glassware under a positive pressure of nitrogen at room temperature unless otherwise noted. CH₂Cl₂ and CH₃CN were dried over CaH₂ and CH₃OH was dried over Mg and distilled prior to their use in reactions. THF was distilled over Na before use. Solvents such as DMF and N(*i*-Pr)₂Et were distilled under reduced pressure. Dimethylaminopyridine (DMAP) was purified by recrystallization from EtOAc and *n*-hexane before use. The eluents for flash chromatography e.g. EtOAc, acetone, and *n*-hexane were of industrial grade and were distilled prior to use; other chromatographic solvents e.g., CH₃OH, CH₂Cl₂ and CHCl₃ were of reagent grade and used without further purification. NMR spectroscopy including ¹H-NMR (500 MHz),¹³C-NMR (125 MHz, DEPT-135) and ¹⁹F-NMR (470 MHz) was performed on a Unity Inova 500 MHz instrument (Varian, USA). Deuterated-solvent CDCl₃ employed for NMR spectroscopy was purchased from Aldrich (St. Louis, MO, USA). Low-Resolution Mass Spectrometry (LRMS) was performed on an ESI-MS spectrometer using a Varian 901-MS Liquid Chromatography Tandem Mass Q-TOF Spectrometer or Quattro Micro LC/MS/MS. High-Resolution Mass Spectrometry (HRMS) was performed using a Varian HPLC (prostar series ESI/APCI) system coupled with a Varian 901-MS (FT-ICR Mass) mass detector and a triple quadrapole instrument. Thin layer chromatography (TLC) was performed with TLC silica gel 60 F₂₅₄ pre-coated glass plates (Machery-Nagel, Dueren, Germany) to monitor the starting materials and products with visualization under UV light (254 nm). Further confirmation was carried out by staining the TLC plates with ninhydrin or ceric ammonium molybdate under heating. Flash chromatography was performed using Silicycle 60 silica gel (70-230 mesh, Quebec city, Quebec, Canada).

Normal phase HPLC comprises an Agilent isocratic 1100 pump, connecting a UVIS detector (254 nm) and a column of ZORBAX SIL (9.4 mm×250 mm, 5 µm). Normal phase HPLC uses a combination of CH₃OH and CH₂Cl₂ 1:19 as the mobile phase at a flow rate of 3 mL/min. A Rheodyne injector with a loop of 0.5 mL was employed.

Boron concentration was determined by ICP-Mass spectrometry using the service by Thermo Fisher Scientific iCAP TQ, Germany.

Radiofluorination was carried out in Nuclear Energy Research Institute at Taoyuan Longtan, Taiwan (China), The hotcell encompases the remote control module and the detailed synthetic procedure is described in sec. 1.5.3. Radioactive compound was purified in NERI through the first HPLC set for initial fractionation including a pump of Eldex Model:2SMP with a 1/8 inch head and a stainless damper and a column ZORBAX SIL (9.4 mm×250 mm, 5 µm) at a flowrate of 3 mL/min. Second HPLC set for quality analysis or purification includes Waters 600 Controller Model Code 6CE, assembled with a pump of Model Code 60F, a UV detector of Waters 2489, a radioactive detector raytest Gabi and the same column ZORBAX SIL (9.4 mm×250 mm, 5 µm) at a flowrate of 1 mL/min, in case of preparation at a flow rate of 3 mL/min.

The PET scanning experiment was performed using SuperArgus PET/CT scanner Inc. Spain at Chinese Taipei Medical University.

The procedure regarding general deprotection of the carboxy group is as follows. To a mixture of TB-5-BTme **2** in 1.5 mL of THF/H₂O 2:1 at 0°C was added LiOH (29 mg, 1.21 mmol, 3 eq). The mixture was stirred at rt for 4 h. In the case of TB-6-BTme 1, a reaction time of 16 h was employed. TLC (CH₃OH/CH₂Cl₂ = 1/19) indicated the consumption of starting material 2 (*R_{f}* = 0.98, 0.76) and formation of the product 4 (*R_{f}* = 0.32, 0.08). After concentration under reduced pressure, the residue was chromatographed with eluents (CH₃OH/CH₂Cl₂ = 1/19) to provide a viscous product TB-5-BT 4 (237 mg, 0.38 mmol) in 92% yield with a melting point ranging from 122 to 124°C. In the case of TB-6-BT 3, purification was performed either through an extraction with column chromatography or treatment with cation exchange resin (H⁺, Dowex) followed by filtration. The desired products TB-6-BT 3 and TB-5-BT 4 were obtained in 73% and 93% yield, respectively. NMR spectra of these deprotected products were measured in CD₃OD. Both of them were unstable in CDCl₃ even in the presence of Ag foil.

### TB-5-BT 4

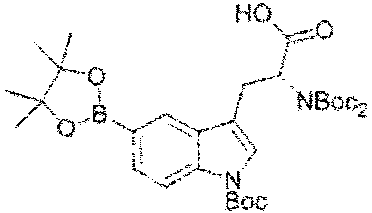

¹H-NMR (500 MHz, CD₃OD) δ 1.29 (s, 18H, N-Boc), 1.35 (s, 12H, Bpin), 1.65 (s, 9H, N-Boc), 3.44-3.46 (m, 2H, H-1'a, H-1'b), 5.18 (dd, *J*_{2',1'b} = 10.0, *J*_{2',1'a} = 6.5 Hz, 1H, H-2' ), 7.39 (s, 1H, H-2), 7.67 (d, *J*_{6,7} = 9.0 Hz, 1H, H-6), 7.98 (s, 1H, H-4), 8.07 (d, *J*_{7,6} = 9.0 Hz, 1H, H-7); ¹³C-NMR (125 MHz, CD₃OD) δ 25.22 (CH₃, Bpin), 26.39 (CH₂, C-1'), 27.98 (CH₃, N-Boc), 28.05 (CH₃, N-Boc), 60.06 (CH, C-2'), 75.84 (C, C-3), 84.31 (C, N-Boc), 85.04 (C, Bpin), 115.49 (CH, C-7), 118.39 (C, C-9), 125.65 (CH, C-6), 127.27 (CH, C-4), 131.66 (CH, C-2), 138.95 (C, C-8), 150.81 (C, N-Boc), 153.19 (C, N-Boc), 173.52 (C, COOH); Analysis for C₃₂H₄₇BN₂O₁₀, calculated for [M+Na]⁺ (m/z) = 653.3221 (100.0%), 654.3255 (34.6%), 652.3258 (24.8%), ESI-Q-TOF HR-ESI-MS found: [M+Na]⁺= 653.3225 (29.8%), 654.3276 (10.0%),652.3285 (6.8%), δ[ppm] = 0.6.

### TB-6-BT 3

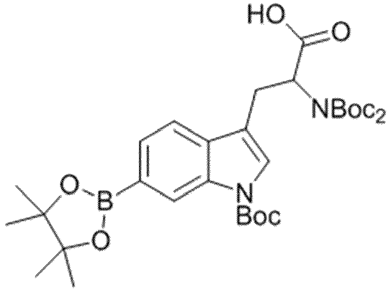

¹H-NMR (500 MHz, CD₃OD) δ 1.28 (s, 18H, N-Boc), 1.36 (s, 12H, Bpin), 1.65-1.67 (m, 9H, N-Boc), 3.38-3.48 (m, 2H, H-1'a, H-1'b), 5.15 (dd, *J*_{2',1'a} = 10.5, *J*_{2',1'b} = 4.5 Hz, 1H, H-2' ), 7.44 (s, 1H, H-2), 7.54 (d, *J*_{5,4} = 8.0 Hz, 1H, H-5), 7.60 (d, *J*_{4,5} = 8.0 Hz, 1H, H-4), 8.53 (s, 1H, H-7); ¹³C-NMR (125 MHz, CD₃OD) δ 25.22 (CH₃, Bpin), 26.35 (CH₂, C-1'), 27.99 (CH₃, N-Boc), 28.39 (CH₃, N-Boc), 59.81 (CH, C-2'), 75.84 (C, N-Boc), 84.29 (C, N-Boc), 85.03 (C, Bpin), 118.22 (C, C-3), 119.46 (CH, C-7), 122.76 (CH, C-4), 126.68 (CH, C-5), 129.55 (CH, C-2), 134.45 (C, C-8), 136.55 (C, C-9), 150.80 (C, N-Boc), 153.28 (C, N-Boc), 173.45 (C, COOH); Analysis for C₃₂H₄₇BN₂O₁₀, calculated for [M+Na]⁺ (m/z) = 653.3221 (100.0%), 654.3255 (34.6%), 652.3258 (24.8%), ESI-Q-TOF HR-ESI-MS found: [M+Na]⁺= 653.3224 (100.0%), 654.3228 (38.6%), 652.3253 (22.4%), δ[ppm] = 0.5.

The procedure regarding Pegylation reaction is as follows. A mixture of TB-6-BT 3 (120 mg, 0.189 mmol) in CH₃CN (3 mL) was equipped with a relatively large stirbar in a two-necked round bottomed flask (50 mL) under protection of N₂. A mixture of hexafluorophosphate benzotriazole tetramethyl uronium (HBTU, 4 eq, 0.756 mmol, 286 mg) and dimethyl amino pyridine (DMAP, 4 eq, 0.756 mmol, 92 mg) in CH₃CN (3 mL) was then added. NEt₃ (100 eq, 5.3 mL) was added and the mixture was stirred for 10 min. Two equivalents of PEG 200 that was codistilled twice using NEt₃ (2 mL) was added per CH₃CN (2 mL). The reaction was allowed for 20-24 h. The whole mixture was then concentrated under reduced pressure. Followed by column chromatography using CH₃OH/CH₂Cl₂ 1:19, the product mixtures with a range of MW distribution of PEG moieties with distributed profile were obtained in a yield of 30%. For the case of PEG200 and PEG1000, a yield of 40-60% was obtained. Some of these chromatographed samples were purified further using normal-phase HPLC with eluents of CH₃OH/CH₂Cl₂ = 1:19. The pegylated product mixtures always colored in pink or purple and smell pleasant.

The preparation process of the N₂O₄ saturated nitric acid (red fuming HNO₃) required for the nitration step is as follows (see FIG. 2). Copper wires from the PC cables cut in small pieces were added to the Erlenmeyer flask. A volume of 30 mL of concentrated yellow-fuming HNO₃ prepared from NH₄NO₃ or NaNO₃ and H₂SO₄ was added. Under stirring and optional warming, the evolving red-fuming gas N₂O₄ was bubbled through the right bubbling flask. When the evolving gas was ceasing (c.a. 5 min), optional water and warming (~60°C) were given to drive the gas evolving. The bubbling was stopped after 20 min through complete bubbling and the solution also turned red-brown. Notice: The N₂O₄-saturated HNO₃ will evolve the N₂O₄ gas from time to time when returning to room temperature. The bottle for storage should not to be capped tightly.

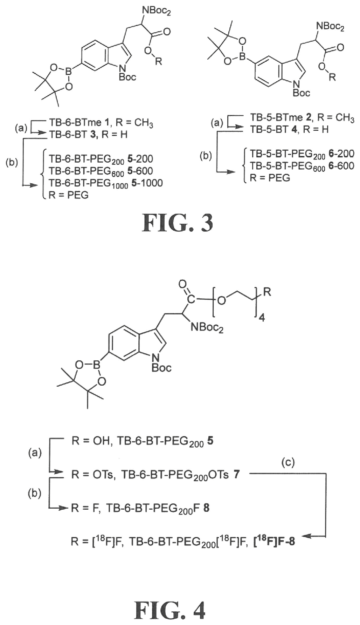

Boronotryptophan compound 1 (TB-6-BTme **1**) could be prepared in a 6-step synthesis via nitration, Boc introduction, reduction, iodination and borylation of the starting material tryptophan. The nitration is optimized by using red-fuming nitric acid prepared by bubbling of N₂O₄ generated through a mixture of the cut cupper wires in the concentrated nitric acid under the addition of H₂O. The brown N₂O₄ saturated nitric acid is used to prepare the desired 6-nitro tryptophan along with a minor 4-nitro isomer in an overall 65% yield. The subsequent deprotection using LiOH (aq) was mild while purification varied by using either direct concentration or extraction followed by chromatography (Scheme **1,** see FIG. 3).

FIG. 3 shows the scheme 1 of boronotryptophan compound 1 (TB-6-BTme 1), in which (a) LiOH, THF/H₂O 2:1, 0 °C, rt for 4-16 h, 73-93% yield (TB-6-BT **3** and TB-5-BT **4**), (b) HBTU (4 eq), DMAP (4 eq), NEt₃ (100 eq), PEG200-1000 (2 eq), 20 h, 30-60% yield (TB-6-BT-PEG₂₀₀ **5**-200, TB-6-BT-PEG₆₀₀ **5**-600, TB-6-BT-PEG₁₀₀₀ **5**-1000, TB-5-BT-PEG₂₀₀ **6**-200, and TB-5-BT-PEG₆₀₀ **6**-600).

Direct fluorination of the terminal hydroxyl group of the PEGylated tryptophan using diethyl amino sulfur trifluoride failed whereas introduction of the tosyl group enabled subsequent fluorination as well as radiofluorination (Scheme 2, see FIG. 4). On the other hand, the solvent condition needs to be optimized is spite of the common CH₃CN for radiofluoride chemistry. In our hands, dimethylforamide sensitized the reaction and a satisfied radiochemical yield (1.7%) (radioactive compound **[¹⁸F]F-8**) was obtained.

FIG. 4 shows the scheme 2 of preparation of the tosylated pegylated TB-6-BT **7**, followed by fluorination and radiofluorination. (a) TsCl, toluene, rt for 3h, 61% yield (TB-6-BT-PEG₂₀₀OTs **7**). (b) KF, DMF, 100 °C for 20 min. (c) H[¹⁸F]F, K₂CO₃, Kryptofix[2,2,2], CH₃CN, 1.7% corrected radiochemical yield (EOB) (radioactive compound **[¹⁸F]F-8**).

PEGylation was firstly conditioned by using benzoic acid. Due to the feature of molecular weight distribution of PEG agent, the product mixture distributes itself and smears through the TLC development rending the column chromatography difficult. Whereas the common PEGylation using excess amount of adduct to overwhelmingly consume the PEG agent, the potential hydrogen bonding through the nucleophilic OH group results in incompleteness. In spite of a common preparation of using DCC as coupling agent and dialysis to separate the PEGylated product from the remained reagents, the result is not satisfied in our hands. For example, ¹H-NMR spectra did not provide a satisfied integral ratio to estimate the unremoved PEG agent. A significant portion of the residual PEG agents remained in the solution can coagulate the PEGylated product. Moreover, due to the overlapping with other reagents in chromatography such as DMAP and HBTU, the purification was cumbersome yet being optimized through a repeated chromatography. Further confirmation of these PEGylated products was accomplished using HPLC and a range of the PEG product mixtures could be fractionated.

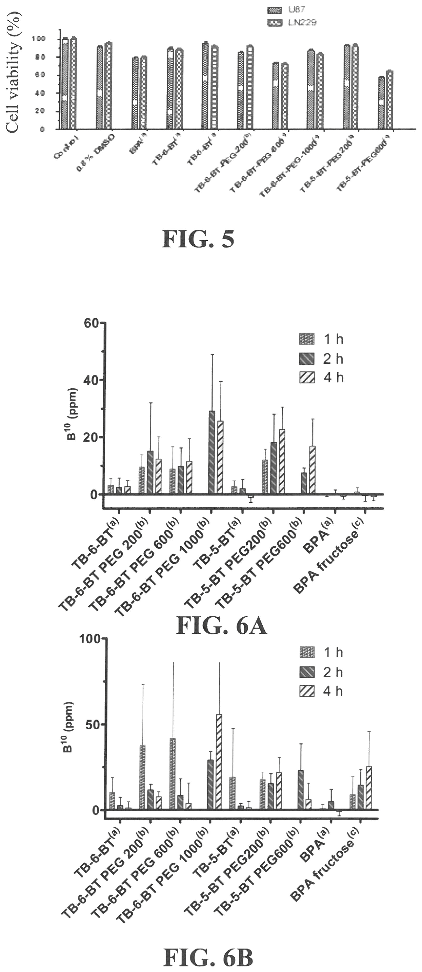

FIG. 5 shows the cell survival assay using different boronotryptophan analogs and controls. Three different concentrations (one concentration for each compound, determined by the compound's water solubility) were specified as (a), (b) and (c) due to different aqueous solubility. (a) 500 µM+0.8% DMSO, (b) 1000 µM+0.8% DMSO and (c) 2000 µM+0.8% DMSO.

The subsequent bioassay can be performed in a low DMSO concentration (0.8%) to decrease the potential toxic side effects (FIG. 5). Through the complexation with fructose, the improved aqueous soluble BPA-fructose conjugate can be increased in double the concentration of these PEGylated compounds (improved water solubility allows for higher concentrations without precipitation) by 482 µM vs. 250 µM.

All these PEGylated boronotryptophan analogs did not show significant toxicity (1 mM) implying sufficient boron dosage to be applicable in BNCT (0.5-1 g/kg).

FIGs. 6A and 6B show cellular accumulation of the boron-containing compounds by using three different concentrations (one concentration for each compound, determined by the compound's water solubility) and specifying as (a) 100 µM + 0.8% DMSO, (b) 250 µM + 0.8% DMSO, and (c) 482 µM + 0% DMSO. The two human glioblastoma cell lines U87 (FIG. 6A) and LN229 (FIG. 6B) were incubated for 1 h, 2 h, and 4 h, respectively.

The uptake assay of these PEGylated boronotryptophan compounds were performed in LAT-1 expressed glioma cells such as human glioblastoma cell lines U87 and LN229 (FIGs. 6A and 6B). The accumulation level of boron atoms of the series of PEGylated boronotryptophan compounds in U87 cells is higher than that of BPA-fructose. Among them, TB-6-BT PEG₁₀₀₀ is most prominent by approaching 30 ppm. The corresponding accumulation in LN229 varies, however; only TB-6-BTPEG₁₀₀₀ and TB-5-BTPEG₂₀₀ shows better results of 50 ppm and 25 ppm, respectively. BPA-fructose shows a marginal uptake in the U87 cell (< 3 ppm) and more uptake in LN229 cell (25 ppm), corresponding to its LAT-1 dependent features. The more LAT-1 expressed LN229 cells are set to be a positive control, nevertheless, U-87 cells are not thought as being LAT-1 independent. An increasing accumulation trend in U87 cells reflects the preferential cell type for these PEGylated boronotryptophan compounds. On the contrary, the highest initial compound uptake in LN229 but gradually pumping out characteristics may suggest the inappropriateness as being the targeting cells. For the feasibility of experimental, TB-6-BTPEG₂₀₀ is selected for radiofluorination for *in vivo* study.

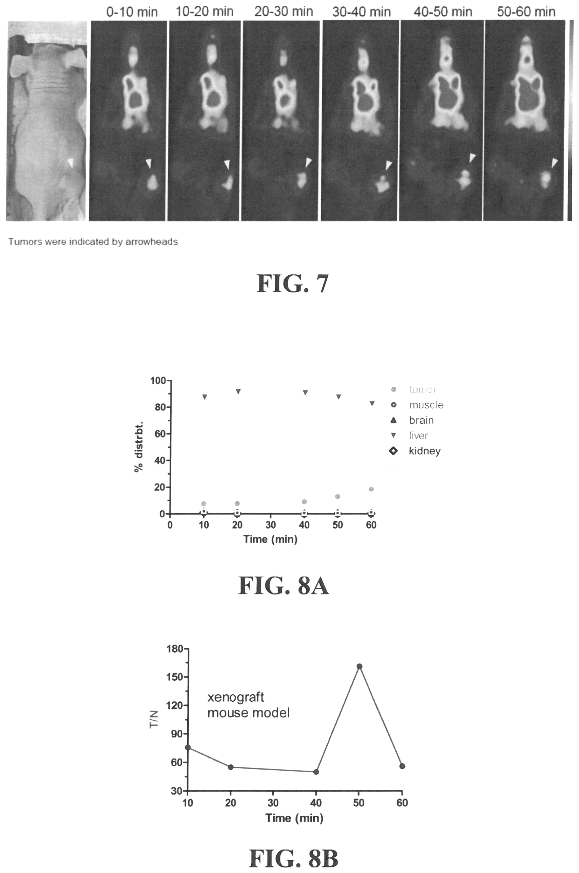

FIG. 7 shows a series of PET imaging taken for temporal distribution of TB-6-BTPEG₂₀₀[¹⁸F]F (**[¹⁸F]F-8)** in the mouse of xenograft model with a tumor cell line of U87, observing the distribution of [¹⁸F]F-8 in tissues or organs.

FIG. 8A shows PET image-derived radioactivity distribution of TB-6-BTPEG₂₀₀[¹⁸F]F (**[¹⁸F]F-8**) in the mouse of xenograft model with a tumor cell line of U87. FIG. 8B shows radioactivity uptake data from the tumor and muscle sites to generate the temporal uptake ratio. The highest tumor to normal tissue uptake ratio 170 (T/N) of **[¹⁸F]F-8** is also noted in 50 min post injection. It means that the high cumulative dose of the tumor is 18% of the drug accumulated in the tumor, and the ratio of high tumor to normal tissue accumulation is about 100-170:1.

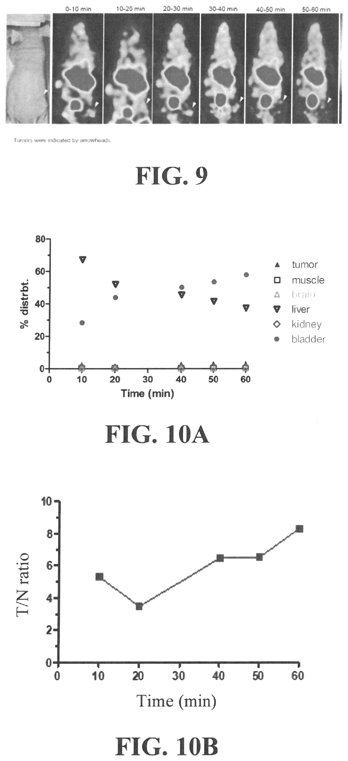

FIG. 9 shows a series of PET imagings taken for temporal distribution of TB-6-BTPEG₂₀₀[¹⁸F]F (**[¹⁸F]F-8**) in the mouse of xenograft model with a tumor cell line of LN229, observing the distribution of [¹⁸F]F-8 in tissues or organs.

FIG. 10A shows PET image-derived radioactivity distribution of TB-6-BTPEG₂₀₀[¹⁸F]F (**[¹⁸F]F-8**) in the mouse of xenograft model with a tumor cell line of LN229. FIG. 10B shows radioactivity uptake data from the tumor and muscle sites to generate the temporal uptake ratio.

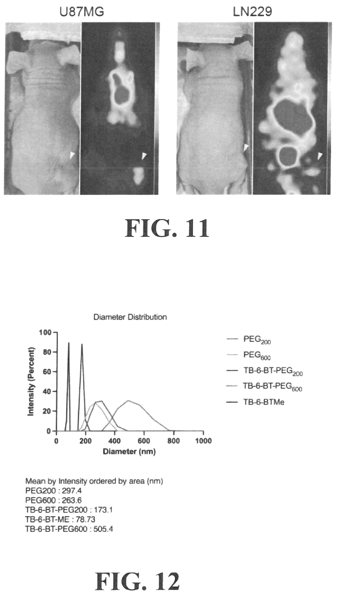

FIG. 11 shows PET imaging for TB-6-BTPEG₂₀₀[¹⁸F]F merged from 0 to 60 min in the mouse of xenograft model with a tumor cell line of U87 and LN229.

The *in vivo* assay of these PEGylated boronotryptophan compounds were realized by using TB-6-BTPEG₂₀₀[¹⁸F]F **[¹⁸F]F-8** to couple with PET (FIGs. 7, 9 and 11). The dynamic PET imagings were spanned from 0 to 60 min for the two cell-line-inoculated xenografted mice and the tumor sites were indicated. The drug concentration of pharmacokinetic analysis can be calculated by quantifying the properties of **¹⁸F**.

On contrary, a low uptake of [¹⁸F]F-8 in LN229 is noted (3% radioactivity) (FIGs. 10A and 10B). An acceptable T/N ratio of 8 at 60 min post injection is shown. The uptake of this PEGylated boronotryptophan compound [¹⁸F]F-8 in brain is probably due to the molecular shape.

FIG. 12 shows size distribution of boronotryptophan derivatives measured by the laser scattering particle size analyzer. Dynamic light scattering results of these PEGylated borono tryptophan compounds show that the average diameter of TB-6-BTPEG₂₀₀ is 173.1±25.0 nm which is larger than its precursor TB-6-BTme 1 (ID = 78.7 nm) but smaller than PEG₂₀₀ (ID = 297.4 nm). An aggregation is significant in the case of TB-6-BTPEG₆₀₀ (ID =505.4 nm) comparing with 263.6 nm of PEG₆₀₀. Because the shorter chain of PEG200 is acting as a substituent in TB-6-BTPEG₂₀₀, the principal structural feature is thus dominated by the tryptophan core shape. The longer PEGylated congener TB-6-BTPEG₆₀₀ shows about twice the size of PEG₆₀₀ indicates a shelling effect through bimolecular coagulation. The satisfied *in-vitro* uptake result of TB-5-BTPEG₂₀₀ might be potential candidate for future radio fluorinating target molecule.

TB-6-BT PEG₂₀₀ 5-200 has the following chemical formula (Ia):

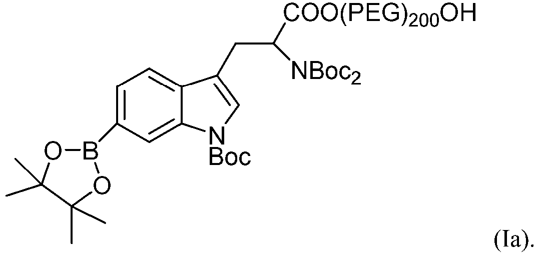

¹H-NMR (500 MHz, CDCl₃) δ 1.31, 1.32, 1.33 (s × 3, 30 H, CO₂C(CH₃)₃, pinacol), 1.62 (s, 9 H, CO₂C(CH₃)₃), 2.78 (s, 5.9 H, OH), 3.33 (dd, *J_{1a',1b'}* = 15.0, *J_{1a',2'}* = 10.0 Hz, 1 H, H-1a'), 3.49 (dd, *J_{1b',1a'}* = 15.0, *J_{1b',2'}* = 5.0 Hz, 1 H, H-1b'), 3.57 (t, *J* = 4.0 Hz, 1.69 H, PEG), 3.62 (s, 5.10 H, PEG), 3.70 (t, *J* = 4.5 Hz, 2.94 H, PEG), 4.23-4.27 (m, 0.81 H, PEG), 4.33-4.38 (m, 0.78 H, PEG), 4.46 (t, *J=* 5.0 Hz, 2.12 H, PEG), 5.20 (dd, *J_{2',1a'}* = 10.0, *J_{2',1b'}* = 5.0 Hz, 1H, H-2'), 7.41 (s, 1H, arom, H-2), 7.50 (dd, *J* = 7.5, 3.5 Hz, 1H, arom, H-4), 7.62 (d, *J* = 8.0 Hz, 1H, arom, H-5) 8.60 (bs,1H, arom, H-7); ¹³C-NMR (125 MHz, CDCl₃) δ 24.88 (CH₃, pinacol), 25.36 (CH₂, C-1'), 27.67 (CH₃, CO₂C(CH₃)₃), 27.77 (CH₃, CO₂C(CH₃)₃), 28.12 (CH₃, CO₂C(CH₃)₃), 58.16 (CH, C-2'); CH₂ (PEG): 61.74, 61.77, 64.61, 68.85, 70.27, 70.34, 70.41, 70.55, 70.65, 70.68, 70.76, 72.48, 72.54; 82.97 (C, CO₂C(CH)₃), 83.34 (C, CO₂C(CH)₃), 83.60 (C, CO₂C(CH)₃), CH (arom): 118.21, 121.75, 125.41, 128.51, C (arom): 116.47, 132.94, 135.26; 149.41 (C, CO₂C(CH₃)₃), 151.79 (C, CO₂C(CH₃)₃), 170.28 (C, CO₂PEG₂₀₀).

TB-6-BT PEG₆₀₀ 5-600 has the following chemical formula (Ib):

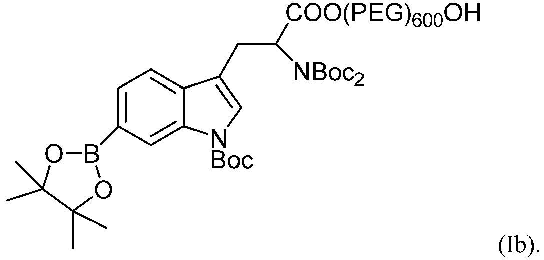

¹H-NMR (500 MHz, CDCl₃) δ 1.31, 1.32 (s × 2, 30 H, CO₂C(CH₃)₃, pinacol), 1.63 (s, 9 H, CO₂C(CH₃)₃), 3.34 (dd, *J_{1a',1b'}* = 15.0, *J_{1a',2'}* = 10.5 Hz, 1 H, H-1a'), 3.57 (dd, *J_{1b',1a'}* = 15.0, *J_{1b',2'}* = 5.0 Hz, 1 H, H-1b'), 3.68-3.71 (m, 32.2 H, PEG), 3.68-3.71 (m, 3.4 H, PEG), 4.22-4.35 (m, 2 H, PEG), 5.19 (dd, *J_{2',1a'}* = 10.5, *J_{2',1b'}* = 5.0 Hz, 1H, H-2'), 7.40 (s, 1H, arom, H-2), 7.49 (d, *J* = 8.0 Hz, 1H, arom, H-4), 7.62 (d, *J* = 8.0 Hz, 1H, arom, H-5) 8.59 (s,1H, arom, H-7); ¹³C-NMR (125 MHz, CDCl₃) δ 24.88 (CH₃, pinacol), 25.35 (CH₂, C-1'), 27.66 (CH₃, CO₂C(CH₃)₃), 27.76 (CH₃, CO₂C(CH₃)₃), 28.11 (CH₃, CO₂C(CH₃)₃), 58.13 (CH, C-2'); CH₂ (PEG): 61.50, 64.57, 68.80, 69.98, 70.26, 70.31, 70.40, 70.47, 70.61, 72.36; 82.96 (C, CO₂C(CH)₃), 83.35 (C, CO₂C(CH)₃), 83.59 (C, CO₂C(CH)₃); CH (arom): 118.17, 121.75, 125.39, 128.50; C (arom): 116.44, 124.70, 132.92, 135.23; 149.39 (C, CO₂C(CH₃)₃), 151.79 (C, CO₂C(CH₃)₃), 170.27 (C, CO₂PEG₆₀₀).

TB-6-BT PEG₁₀₀₀ **5**-1000 has the following chemical formula (Ic):

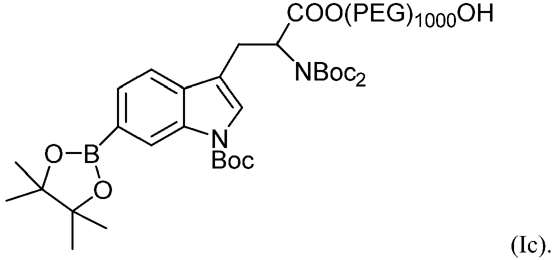

¹H-NMR (500 MHz, CDCl₃) δ 1.26, 1.28, 1.30 (s × 2, 33 H, CO₂C(CH₃)₃, pinacol), 1.58 (s, 13 H, CO₂C(CH₃)₃), 1.75 (bs, 6 H, H₂O), 2.73 (s, 1.54 H, OH), 3.29 (dd, *J_{1a',1b'}* = 15.0, *J_{1a',2'}* = 10.0 Hz, 1.44 H, H-1a'), 3.44 (dd, *J_{1b',1a'}* = 15.0, *J_{1b',2'}* = 5.0 Hz, 2.09 H, H-1b'), 3.57 (s, 63.01 H, PEG), 3.64-3.67 (m, 3.54 H, PEG), 4.19-4.30 (m, 2.64 H, PEG), 5.14 (dd, *J_{2',1a'}* = 10.0, *J_{2',1b'}* = 5.0 Hz, 1H, H-2'), 7.36 (s, 1H, arom, H-2), 7.44 (d, *J* = 7.5 Hz, 1H, arom, H-4), 7.58 (d, *J* = 7.5 Hz, 1H, arom, H-5) 8.55 (s,1H, arom, H-7).; ¹³C-NMR (125 MHz, CDCl₃) δ 24.83 (CH₃, pinacol), 25.30 (CH₂, C-1'), 27.71 (CH₃, CO₂C(CH₃)₃), 28.06 (CH₃, CO₂C(CH₃)₃), 28.26 (CH₃, CO₂C(CH₃)₃), 29.61 (CH₃, CO₂C(CH₃)₃), 58.08 (CH, C-2'); CH₂ (PEG): 61.40, 64.53, 68.75, 69.88, 70.16, 70.25, 70.41, 70.56, 72.25; 82.91 (C, CO₂C(CH)₃), 83.30 (C, CO₂C(CH)₃), 83.55 (C, CO₂C(CH)₃), 83.60 (C, CO₂C(CH)₃); CH (arom): 118.13, 121.71, 125.35, 128.45; C (arom): 116.40, 132.87, 135.19; 149.34 (C, CO₂C(CH₃)₃), 151.74 (C, CO₂C(CH₃)₃), 170.22 (C, CO₂PEG₁₀₀₀).

TB-5-BT PEG₂₀₀ 6-200 has the following chemical formula (IIa):

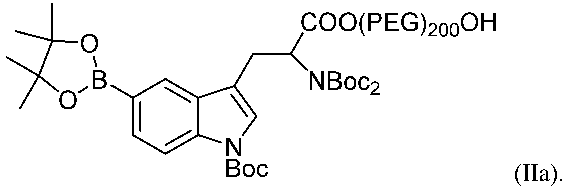

¹H-NMR (500 MHz, CDCl₃) δ 1.31, 1.33 (s × 2, 30 H, CO₂C(CH₃)₃, pinacol), 1.62 (s, 9 H, CO₂C(CH₃)₃), 3.36 (dd, *J_{1a',1b'}* = 15.0, *J_{1a',2'}* = 10.5 Hz, 1 H, H-1a'), 3.51 (dd, *J_{1b',1a'}* = 15.0, *J_{1b',2'}* = 4.0 Hz, 1 H, H-1b'), 3.56-3.72 (m, *J* = 4.0 Hz, 16 H, PEG), 4.24-4.28 (m, 1 H, PEG), 4.34-4.40 (m, 1 H, PEG), 4.46 (t, *J* = 5.0 Hz, 2.12 H, PEG), 5.23 (dd, *J_{2',1a'}* = 10.5, *J_{2',1b'}* = 4.0 Hz, 1H, H-2), 7.34 (s, 1H, arom, H-2), 7.72 (dd, *J* = 8.5 Hz, 1H, arom, H-4), 7.97 (s, 1H, arom, H-5) 8.08 (d, *J* = 7.5 Hz, 1H, arom, H-7); ¹³C-NMR (125 MHz, CDCl₃) δ 24.86 (CH₃, pinacol), 24.90 (CH₃, pinacol), 25.28 (CH₂, C-1'), 27.76 (CH₃, CO₂C(CH₃)₃), 28.15 (CH₃, CO₂C(CH₃)₃), 58.10 (CH, C-2'); CH₂ (PEG): 61.74, 64.64, 68.84, 70.27, 70.35, 70.41, 70.52, 70.55, 70.64, 70.69, 70.80, 72.49, 72.54; 82.92 (C, CO₂C(CH)₃), 82.98 (C, CO₂C(CH)₃), 83.51 (C, CO₂C(CH)₃), 83.64 (C, CO₂C(CH)₃), CH (arom): 114.49, 124.40, 126.02, 130.86, C (arom): 116.68, 130.02, 137.62; 149.46 (C, CO₂C(CH₃)₃), 151.63 (C, CO₂C(CH₃)₃), 170.33 (C, CO₂PEG₂₀₀).

TB-5-BT PEG₆₀₀ **6**-600 has the following chemical formula (IIb):

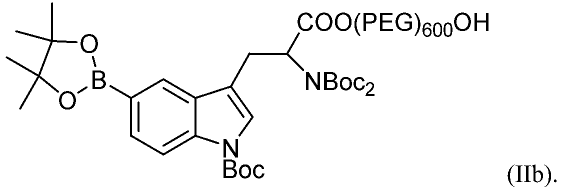

¹H-NMR (500 MHz, CDCl₃) δ 1.30, 1.33 (s × 2, 30 H, CO₂C(CH₃)₃, pinacol), 1.62 (s, 9 H, CO₂C(CH₃)₃), 3.34 (dd, *J_{1a',1b'}* = 14.5, *J_{1a',2'}* = 10.5 Hz, 1 H, H-1a'), 3.57 (dd, *J_{1b',1a'}* = 15.0, *J_{1b',2'}* = 5.0 Hz, 1 H, H-1b'), 3.68-3.71 (m, 32.2 H, PEG), 3.68-3.71 (m, 3.4 H, PEG), 4.22-4.35 (m, 2 H, PEG), 5.19 (dd, *J_{2',1a'}* = 10.5, *J_{2',1b'}* = 5.0 Hz, 1H, H-2'), 7.40 (s, 1H, arom, H-2), 7.49 (d, *J* = 8.0 Hz, 1H, arom, H-4), 7.62 (d, *J* = 8.0 Hz, 1H, arom, H-5) 8.59 (s,1H, arom, H-7); ¹³C-NMR (125 MHz, CDCl₃) δ 24.87 (CH₃, pinacol), 24.90 (CH₃, pinacol), 25.28 (CH₂, C-1'), 27.77 (CH₃, CO₂C(CH₃)₃), 28.16 (CH₃, CO₂C(CH₃)₃) , 58.09 (CH, C-2'); CH₂ (PEG): 61.66, 64.61, 68.81, 70.22, 70.67, 72.55; 82.91 (C, CO₂C(CH)₃), 83.51 (C, CO₂C(CH)₃), 83.63 (C, CO₂C(CH)₃); CH (arom): 114.49, 124.39, 126.01, 130.86; C (arom): 116.68, 130.02, 137.61; 149.46 (C, CO₂C(CH₃)₃), 151.63 (C, CO₂C(CH₃)₃), 170.32 (C, CO₂PEG₆₀₀).

TB-6-BT PEG₂₀₀-OTs **7** has the following chemical formula (IIIa):

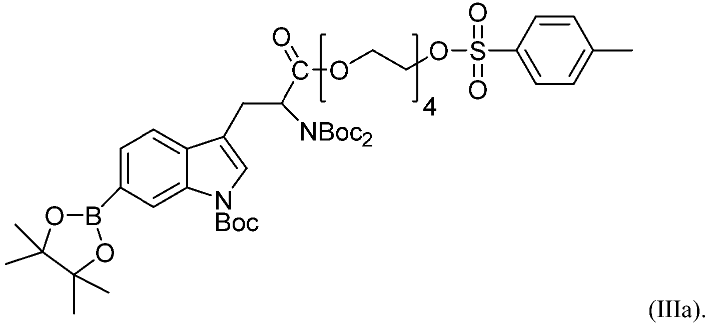

Starting material TB-6-BT-PEG₂₀₀ (160 mg, 216 mmol, 1 eq) and TsCl (60 mg, 0.324 mmol, 1.5 eq) were individually treated with toluene (3 mL x 3) to distill H₂O. They were transferred to a two-necked round-bottomed flask via CH₂Cl₂ (5 mL) under N₂, sequentially. Followed by addition of Et₃N (0.06 mL, 0.44 mmol, 2 eq), the mixture was monitored with TLC (CH₃OH / CH₂Cl₂ = 1 / 19). The consumption of molecular weight-distributed starting material profiles (R*_{f}* = 0.32~0.61) and formation of the molecular weight-distributed product profiles (R*_{f}* = 0.83~0.96) was observed. The mixture post 3 h was concentrated under reduced pressure. Followed by flash chromatography using CH₃OH / CH₂Cl₂ = 1 : 79, a pale yellow oil with a smell of wooden shavings was obtained in 61 % yield (120 mg).

¹H-NMR (500MHz, CDCl₃) δ 1.31 (s, 27 H, CO₂C(CH₃)₃) , 1.33, 1.62 (s, 12.0 H, CO₂C(CH₃)₃), 2.41 (s, 3.0 H, -CH₃),3.33 (dd, *J_{1a',1b'}* = *15.0, J_{1a',2'}* = 4.5 Hz, 1 H, H-1a'), 3.48 (dd, *J_{1b',1a'}* = 15.0, *J_{1b',2'}* = 10.0 Hz, 1 H, H-1b'), 3.54-3.55 (m, 3.7 H, PEG) , 3.58-3.59 (m, 3.3 H, PEG),3.64-3.66 (m, 4.2 H, PEG) , 4.12-4.14 (m, 2.1 H, PEG),4.23-4.25 (m, 1.3 H, PEG), 4.24-4.32 (m, 1.1 H, PEG), 5.20 (dd, *J_{2',1a'}* = 10.0, *J_{2',1b'}* = 5.0 Hz, 1H, H-2'), 7.41 (s, 1H,), 7.48 (dd, *J* = 7.5, 3.7 Hz, 1H), 7.62 (d, *J* = 8.0 Hz, 1H) 8.60 (s,1H). ¹³C-NMR (125MHz, CDCl₃) δ 24.79 (CH₃, CH₃CCH₃), 24.80 (CH₃, CH₃CCH₃) , 25.28 (CH₂, C-1'), 27.59 (CH₃, CO₂C(CH₃)₃), 27.68 (CH₃, CO₂C(CH₃)₃) 28.03 (CH₃, CO₂C(CH₃)₃) , 53.36 (CH, C-2'), 58.06 (CH, C-2'); CH₂ (PEG): 61.74, 61.77, 64.61, 68.85, 70.27, 70.34, 70.41, 70.55, 70.65, 70.68, 70.76, 72.48, 72.54; 82.97 (C, CO₂C(CH)₃), 83.34 (C, CO₂C(CH)₃), 83.60 (C, CO₂C(CH)₃), CH (arom): 118.21, 121.75, 125.41, 128.51, C (arom): 116.47, 132.94, 135.26; 149.41 (C, CO₂C(CH₃)₃), 151.79 (C, CO₂C(CH₃)₃), 170.28 (C, CO₂ PEG₂₀₀).

TB-6-BT PEG200-F **8** has the following chemical formula (IIIb):

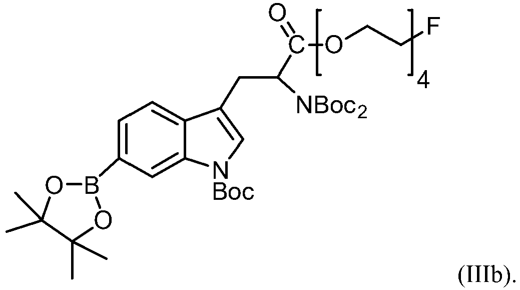

KF (14.5 mg, 0.25 mmol, 5 eq) in a two-necked round-bottomed flask was dried in an oven at 120 °C for 24 h. Starting material TB-6-BT-PEG₂₀₀-OTs (50 mg, 0.05 mmol, 1 eq) was distilled with toluene (3 mL x 3). TB-6-BT-PEG₂₀₀-OTs was transferred with DMF (1.5 mL) to a two-necked round-bottomed flask. Kryptofix [2,2,2] (26 mg, 0.07 mmol, 1.4 eq) was then added. The mixture was stirred at 100 °C for 20 min. Followed by flash chromatography with eluents of CH₃OH / CH₂Cl₂ = 1 / 19, a pale yellow oily liquid was obtained in 51% yield (48 mg). The ¹H-NMR indicates the presence of a hydrolyzed analog constituting 50% portion.

¹H-NMR (500MHz, CDCl₃) δ 1.31 (s, 27 H, CO₂C(CH₃)₃) , 1.33, 1.62 (s, 12.0 H, CO₂C(CH₃)₃) ,3.33 (dd, *J_{1a',1b'}* = 15.0, *J_{1a',2'}* = 4.5 Hz, 2 H, H-1a'), 3.48 (dd, *J_{1b',1a'}* = *15.0, J_{1b',2'}* = 10.0 Hz, 2 H, H-1b'), 3.57-3.7 (m, 21.0 H, PEG) , 4.23-4.34 (m, 3.8 H, PEG), 4.47-4.57 (2.0 H, PEG), 5.20 (dd, *J_{2',1a'}* = 10.0, *J_{2',1b'}* = 5.0 Hz, 1.6H, -CH), 7.41 (s, 1H,), 7.49 (dd, *J* = 7.5 Hz, 1.4H), 7.63 (d, *J* = 8.0 Hz, 1.4H) 8.60 (s,1H)
¹⁹F-NMR (470MHz, CDCl₃)
δ -222.93

### Preparation of pegylated benzoic acid esters

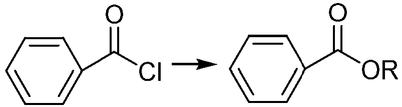

Bz-PEG₂₀₀ **7**-200
Bz-PEG₄₀₀ **7**-400
Bz-PEG₆₀₀ **7**-600
Bz-PEG₁₀₀₀ **7**-1000
**R = PEG**

Benzoyl-PEG600-OH as an example. To a mixture of PEG-600 (909 µL, 1.7 mmol, 1 eq) in a round-bottomed flask (50 mL) was added Et₃N (2 mL) and CH₂Cl₂ (2 mL), sequentially, for coevaporation. The mixture was then protected with N2, followed by addition of DMAP (415 mg, 3.4 mmol, 2 eq), Et₃N (0.2 mL, 1.4 mmol, 0.8 eq). The mixture was brought to homogeneous using CH₂Cl₂ (5 mL). A solution of benzoyl chloride (454 µL, 3.91 mmol, 2.3 eq) in CH₂Cl₂ (5 mL) was added. The white cloudy mixture post 90 min was monitored with TLC. (CH₃OH/CH₂Cl₂ = 0.8 : 9.2). A major product mixture with less polar part (R*_{f}* = 0.90~0.65) and a minor product with more polar part (R*_{f}* = 0.56) was observed. It was concentrated under reduced pressure post 2.5 h of the reaction. The snow-white crude mixture (2.54 g) was chromatographed using a gradient mode of CH₃OH/CH₂Cl₂ = 1/32-1/19 to give the major monobenzoylated product in 50% yield (1.3 g) and the minor dibenzoylated product in 2% yield (73 mg). The two products were confirmed to be the monobenzoylated PEG-OH and dibenzoylated PEG. In fact, the less polar product is the monobenzoylated benzoyl-PEG600-OH. The more polar one is the dibenzoylated product.

Benzoyl PEG200 **9**-200 has the following chemical formula (IV):

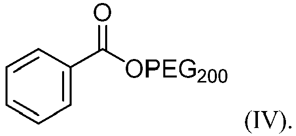

¹H-NMR (500 MHz, CDCl₃) δ 1.63 (s, 1H, OH), 3.64-3.72 (m, 4.60 H, PEG), 3.81-3.85 (m, 2.33 H, PEG), 4.64 (t, *J* = 5.0 Hz, 2.28 H, PEG), 7.43 (t, *J* = 7.5 Hz, 2H, arom-H), 7.55 (t, *J* = 7.5 Hz , 1H, arom-H), 8.05 (d, *J* = 7.5 Hz , 2H, arom-H); ¹³C-NMR (125 MHz, CDCl₃) δ CH₂ (PEG): 64.07, 69.19, 69.26, 70.57, 70.61, 70.65, 70.68, CH (arom): 128.28, 129.63, 132.92, C (arom): 130.09, CO (ester): 166.49.

Benzoyl PEG400 **9**-400 has the following chemical formula (V):

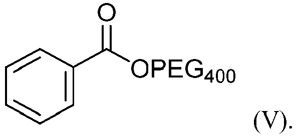

¹H-NMR (500 MHz, CDCl₃) δ 3.60-3.62 (m, 7.32 H, PEG), 3.63-3.65 (m, 2.48 H, PEG), 3.67-3.69 (m, 2.20 H, PEG), 3.81 (t, *J* = 5.0 Hz, 2.18 H, PEG), 4.45 (t, *J* = 5.0 Hz, 2.15 H, PEG), 7.41 (t, *J* = 7.5 Hz, 2.13 H, arom-H, one site + two sites), 7.45 (t, *J* = 7.5 Hz, 2H, arom-H, two sites), 7.53 (t, *J* = 7.5 Hz, 1.02 H, arom-H, one site + two sites), 7.58 (t, *J* = 7.5 Hz, 0.18 H, arom-H, two sites) , 8.03 (d, *J* = 7.5 Hz, 2.00 H, arom-H, one site + two sites), 8.08 (d, *J* = 7.5 Hz , 0.35 H, arom-H, two sites); ¹³C-NMR (125 MHz, CDCl₃) δ CH₂ (PEG): 64.07, 69.16, 70.49, 70.54, 70.57, 70.63, CH (arom): 128.26, 128.33, 129.61, 130.00, C (arom): 130.06, CO (ester): 166.48.

Benzoyl PEG600 **9**-600 has the following chemical formula (VI):

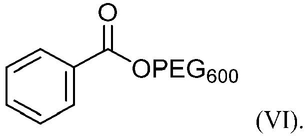

¹H-NMR (500 MHz, CDCl₃) δ 2.31 (s, 1.2 H, OH), 3.61 (s, 18.62 H, PEG), 3.63-3.65 (m, 2.44 H, PEG), 3.67-3.68 (m, 2.18 H, PEG), 3.81 (t, *J* = 5.0 Hz, 2.16 H, PEG), 4.45 (t, *J* = 5.0 Hz, 2.11 H, PEG), 7.41 (t, *J* = 7.5 Hz, 2.05 H, arom-H), 7.53 (t, *J* = 7.5 Hz , 1.01 H, arom-H), 8.03 (d, *J* = 7.5 Hz , 2.00 H, arom-H); ¹³C-NMR (125 MHz, CDCl₃) δ CH₂ (PEG): 64.06, 69.15, 70.49, 70.54, 70.57, 70.63, CH (arom): 128.25, 129.60, 132.89, C (arom): 130.06, CO (ester): 166.45.

Benzoyl PEG1000 **9**-1000 has the following chemical formula (VII):

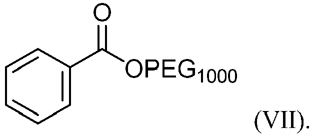

¹H-NMR (500 MHz, CDCl₃) δ 2.21 (bs, 4.58 H, OH), 3.62 (s, 31.95 H, PEG), 3.64-3.66 (m, 2.66 H, PEG), 3.68-3.69 (m, 2.30 H, PEG), 3.82 (t, *J* = 5.0 Hz, 2.16 H, PEG), 4.46 (t, *J* = 5.0 Hz, 2.12 H, PEG), 7.42 (t, *J* = 7.5 Hz, 2.12 H, arom-H), 7.45 (t, *J* = 7.5 Hz, 0.28 H, arom-H), 7.54 (t, *J* = 7.5 Hz , 1.02 H, arom-H), 7.58 (t, *J* = 7.5 Hz, 0.18 H, arom-H), 8.03 (d, *J* = 7.5 Hz, 2.00 H, arom-H), 8.06 (d, *J* = 7.5 Hz, 0.33 H, arom-H); ¹³C-NMR (125 MHz, CDCl₃) δ CH₂ (PEG): 64.04, 69.13, 70.46, 70.51, 70.54, 70.60, CH (arom): 128.24, 129.58, 129.88, 132.88, 133.01, C (arom): 130.03, CO (ester): 166.44.

### Size-distribution measurement of pegylated borono tryptophan derivatives

The samples TB-6-BTMe, TB-6-BT-PEG₂₀₀, TB-6-BT-PEG₆₀₀, PEG₂₀₀ and PEG₆₀₀ were each taken 1 mg for dissolving in CH₃CH₂OH in a vial (3 mL) followed by ultrasonic vibration for 5 min. The particle size measurement was carried out using Zetasizer Ultra-Red mediating through a laser irradiation to extract the information of the diffraction patterns. The data was manipulated by numerical analysis through the software of manufacturer. The size distribution was graphed using GraphPad Prism 5 software.

### Bioassay

Due to the sticky features of these pegylated tryptophan compounds, all the samples were initially weighed and treated with EtOH (20 µL) and ddH₂O (200 µL) followed by lyophilization. The lyophilized products were dissolved in an aqueous solution containing DMSO (13.6% v/v) serving as a stock solution (8.2-33 mM). Followed by a series of dilution to the desired concentration, the subsequent biological analyses were performed.

The procedure regarding *in vitro* cytotoxicity is as follows. The cytotoxicity of tryptophan conjugates against U87 and LN229 cells was measured in a monolayer culture. Cells were plated in 96-well plates (5000 cells/100 µL per well) to allow for attachment. Owning to the advantages brought about by these pegylated compounds with respect to insignificant toxicity and enhancing solubility, the most concentrated formulation includes TB-6-BT-PEG₆₀₀, TB-6-BT-PEG₁₀₀₀, TB-5-BT-PEG₆₀₀ and TB-5-BT-PEG₁₀₀₀ in 2 mM. The moderate aqueous solubility of TB-6-BT-PEG₂₀₀ and TB-5-BT-PEG₂₀₀ were prepared in 1 mM and the least aqueous soluble TB-6-BT, TB-5-BT and BPA were prepared in 0.5 mM. The above mentioned cell-planted medium (100 µM) was removed and replenished by the same volume of corresponding medium containing the pegylated tryptophan derivatives and BPA to give a final mixture containing 0.8% DMSO. After 24 h, cell viability was determined in all wells using an MTT assay and a microplate reader (Spectramax 190, Molecular Devices Corp., Sunnyvale, CA). The medium was replenished by 10% MTT agents (100 µL) and incubated for 4 h. After replacing by DMSO (100 µL), the plate was measured at a λ_{abs} = 570 nm. The fraction of cells surviving was calculated by dividing mean optical density produced by treated cells by the mean optical density from untreated control cells.

The procedure regarding cell accumulation assay is as follows. The two cell lines U87 and LN229 were seeded in a petri dish (ID: 6 cm) containing a medium of 2 mL in a concentration of 2.5 × 10⁵cells/well and 5 × 10⁵ cells/well, respectively. After adhesion to the inner wall for 24 h, the medium was replaced by a new medium containing tryptophan derivatives and BPA (50 µM, 10 mL). The supernatant at each time point of incubation for 0.05, 1, 2, and 4 h was removed and the cells were washed with a PBS solution (5 mL) two times. The residual cell mixture was cleaved using a mixture of trypsin and EDTA (2 mL) for 5 min. The mixture of cell pellets was washed with medium (5 mL) for three times followed by transferring to a centrifuge tube for centrifugation at 1200 rpm for 5 min. The supernatants were removed followed by replenishment of 1 mL of medium for dissolution. An aliquot of 20 µL was mixed with trypan blue (20 µL) and 10 µL of which was drawn for cellular counting. The centrifuge tube was centrifuged again. The supernatant was removed and the precipitate was treated with 70%Wt. HNO_{3(aq)} (75 µL) for digestion for 48h. Additional distilled H₂O (1.42 mL) was added to give a concentration of 3.5% HNO₃(aq) in 1.5 mL. The samples were analyzed for the concentration of born using inductively coupled plasma mass spectrometry (ICP-MS). All the data were obtained in triplicate. The detection limit of the instrument is 0.399 ppb.

The concentration of ¹⁰B atoms was calculated based on the following equation: $\text{μ} {\text{g}}^{10} \text{B/g} = \frac{{\text{ppb}}^{11} \text{B} \times {10}^{- 9} \times 1.5 \left(\text{mL}\right) \times 1 \left(\text{g/mL}\right) \times 0.2 \left({\text{g of}}^{10} {\text{B/g of}}^{11} \text{B}\right) \times {10}^{6} \left(\text{μ} \text{g/g}\right)}{\text{N cell} \times {\text{10}}^{- 9} \left(\text{g/cell}\right)}$

### Radiochemistry

The procedure regarding activation of K⁺kryptofix[2,2,2][¹⁸F]F⁻ is as follows. The purchased aqueous solution of H[¹⁸F]F (262 mCi) was trapped in QMA-Light Sep Park anionic exchange cartridge. A mixture containing K₂CO₃ (5.8 mg) and Kryptofix [2,2,2] (10.4 mg) in a solvent combination of CH₃CN (1 mL) and H₂O (0.5 mL) was used to elute the trapped [¹⁸F]F⁻ to give radiofluoride in > 90% yield (262 mCi). The mixture was heated to 95°C under purging with N₂ gas for 3 min. After cooling to 50°C, the radiochemical activity was 246 mCi. Repeating the procedure by adding CH₃CN (1 mL), heating to 95°C and N₂ gas purging as well as cooling to 50°C, a radioactivity of 208 mCi was obtained.

The procedure regarding rafiofluorination is as follows. The tosylate TB-6-BT-PEG₂₀₀-OTs (20 mg) dissolving in DMF (1 mL) was transferred to the reaction vessel as mentioned above. After heating to 100°C and reacting for 10 min, the vessel pressure was 188 kpa and the radioactivity was 248 mCi. Followed by cooling down to 50°C, the vessel returned to normal pressure. The mixture was eluted through Al₂O₃ cartridge and washed with CH₃CN to give the eluent with a radioactivity of 8.79 mCi along with an activity of 197 mCi remained over the solid support. The mixture was dried at 55 °C and dissolved in CH₃OH/CH₂Cl₂ 1:49 for fractionation with HPLC with eluent CH₃OH/CH₂Cl₂ 1:49 at a flow rate of 3 mL/min to give the product solution with a radioactivity of 3.11 mCi. The correctd radiochemical yield of 1.7% was obtained after 1-h synthesis (EOB). Followed by subsequent concentration and further dryness, the sample was submitted to animal PET imaging study. An analytic amount of sample was further submitted to an analytic HPLC. The injections were formulated in 10% DMSO and saline with an activity of greater than 11 µCi for PET scannings of the two tumoral mice.

The procedure regarding positron emission tomography (PET) study is as follows. All *in vivo* experiments were conducted in compliance with the NHMRC Taiwan (China) Code of Practice for the care and use of animals for scientific purposes. Animal Use Protocol No. 111022 of Nuclear Energy Research Institute was approved before performing the experiment. The rats were housed under fixed environmental conditions and had free access to food and water throughout the experimental period.

*In vivo* PET studies of [¹⁸F]F-TB-6-BT-PEG₂₀₀ [¹⁸F]F-8 were performed using a LN229 tumor-bearing mouse (n = 1, 6 th week post-administration) along with a U87MG tumor-bearing mouse (n = 1, 6th week post-administration).

For the dynamic PET study of [¹⁸F]F-TB-6-BT-PEG₂₀₀ [¹⁸F]F-8, mice were first anesthetized with 1-2% isoflurane and the tumor was positioned on the center of the field of view. After injection of 1.0 ± 0.6 MBq/0.1 mL of [¹⁸F]F-TB-6-BT-PEG₂₀₀ [¹⁸F]F-8 via the tail vein, a 1-h dynamic PET scan was performed to collect 6 ×10-min frames for mice. The 6 × 10-min frames were either analyzed directly or binned together to obtain a 0-60 min scanning set.

The 6 frames were used to plot activity-time curves with the regions of interest (ROIs) covering either the tumor sites or the adjacent normal regions vs. the time course. The binning data sets from frame 4 to frame 6 could additionally serve as one static image frame (0-60 min) in order to compare with other static scanning data. For the group of [¹⁸F]F-TB-6-BT-PEG₂₀₀, the static imaging mode that collected the imaging data over 0-60 min postinjection was used for mice.

Taken together, the PET studies encompassed two tumor-bearing mice. A PET scanner (SuperArgus PET Inc., Spain) was used for the small animal PET scanning study. The images obtained were reconstructed using 2-D ordered subset expectation maximization (OSEM 2D) and processed by PMOD 3.2 imaging analysis software. ROIs were drawn over the tumor with a threshold (maximum intensity minus minimum intensity) × 50% and over the liver, kidney and muslce region. The average intensity in the ROIs (kBq/cc) was measured.

### Partition coefficient determination of [¹⁸F]F-TB-6-BT-PEG₂₀₀ [¹⁸F]F-8

The salt Na₃PO₄ (409 mg, 2.5 mmol) dissolving in a volumetric flask (50 mL) making a solution in 50 mM was titrated with a 100 fold-diluted H₃PO₄ solution until pH 7.4. An aliquot of 3 mL of Na₃PO₄ (aq) and *n*-octanol was mixed and stirred in round-bottomed flask (10 mL) for 10 min. The radiotracer [¹⁸F]F-TB-6-BT-PEG₂₀₀ was added and the stirring lasted for 20 min. An aliquot of 0.2 mL of the organic and aqueous layer, respectively, was pipetted to a test tube in quadruplicate. A volume of 1 mL of CH₃CH₂OH was then added. The radioactivity was then measured using wizard2^{®} automatic gamma counters. A logarithm was taken for the division of the counts of organic layer by that of the aqueous layer.

### Physical properties and description

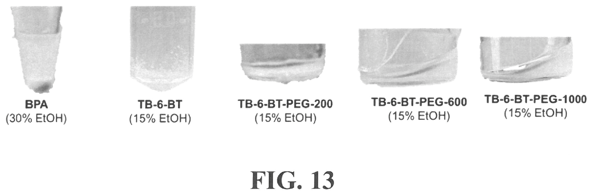

FIG. 13 shows comparison of the aqueous solubility of the pegylated tryptophan analogs. Table 1 describes the aqueous solubility of the pegylated boronotryptophan compounds. As indicated by the small logP value of 0.65±0.04, the lower lipophilicity of [¹⁸F]F-8 has improved the aqueous solubility.

**Table 1**

| Compound ID | Quantity (mg) | solvents | solubility |
|---|---|---|---|
| BPA | 9 | 20 µL EtOH +100 µL H₂O (15% EtOH) | insoluble |
| | | 40 µL EtOH +100 µL H₂O (30% EtOH) | insoluble |
| TB-6-BT | 2 | 8 µL EtOH +40 µL H₂O (15% EtOH) | insoluble |
| TB-6-BTPEG₂₀₀ | 6 | 10 µL EtOH +60 µL H₂O (15% EtOH) | Slightly soluble, gel-like |
| TB-6-BT PEG₆₀₀ | 4 | 8 µL EtOH +40 µL H₂O (15% EtOH) | Slightly cloudy, almost soluble |
| TB-6-BT-PEG₁₀₀₀ | 10 | 100 µL H₂O (0 % EtOH) | soluble |

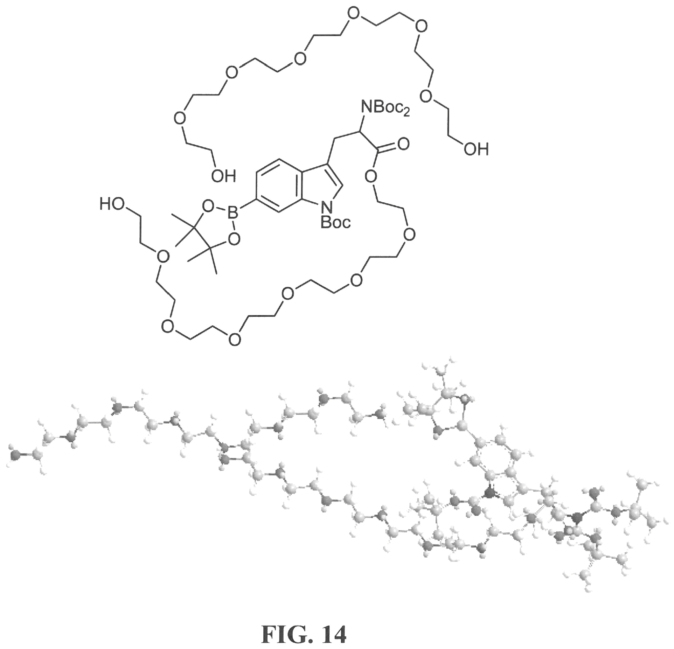

FIG. 14 shows proposed coagulation of the pegylated boronotryptophan analogs.

The current study provided a new class of boronotryptophan compounds through PEGylation to improve their aqueous solubility for serving as injection formulation, whereas the Boc-protected core structure can still be able to cross the cell membrane. Due to the labile ester group, once the compound has circulated through the blood stream, the core structure of boronotryptophan can be released through ester hydrolysis by lipase. Such a prodrug concept has shown a positive result from a significant accumulation of [¹⁸F]F-8 in U87 xenografted tumor mouse. Also a high T/N ratio of 170 is addressed.

As being one of the rare reports of imaging of PEGylated compounds so far, the [¹⁸F]F-PEGylated boronotryptophan compound in PET application with an acceptable radiochemical activity (<100 µCi) has been used to generate the *in-vivo* image for quickly bridging a compound from fresh bench synthesis to an initial *in-vivo* evaluation of its efficacy.

In summary, the effects of the PEGylated boronotryptophan derivative and the pharmaceutical composition comprising the PEGylated boronotryptophan derivative of the present invention are as follows: The results illustrated in the above examples achieve the effects on treating tumor (e.g., brain tumor) and for positron emission tomography (PET) scanning for assessing pharmacokinetics for boron neutron capture therapy (BNCT) of tumors and quantifying drug concentrations to calculate pharmacokinetics.

The above description is for illustrative purposes only and is not intended to be limiting. Any equivalent modification or change made to the present invention without departing from the spirit and scope of the present invention shall be included in the appended claims.

## Claims

1. A PEGylated boronotryptophan derivative, having the following chemical formula (I): 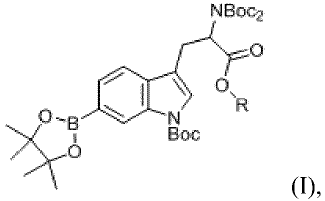 wherein R is polyethylene glycol (PEG).

2. The PEGylated boronotryptophan derivative of claim 1, wherein the PEG is PEG₂₀₀, and when the PEG is PEG₂₀₀, the PEGylated borotryptophan derivative has the following chemical formula (Ia):

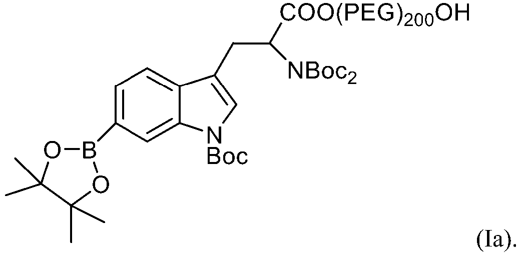

3. The PEGylated boronotryptophan derivative of claim 1, wherein the PEG is PEG₆₀₀, and when the PEG is PEG₆₀₀, the PEGylated borotryptophan derivative has the following chemical formula (Ib):

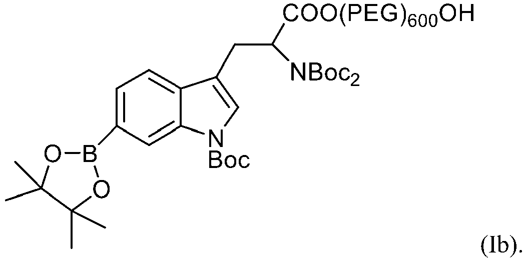

4. The PEGylated boronotryptophan derivative of claim 1, wherein the PEG is PEG₁₀₀₀, and when the PEG is PEG₁₀₀₀, the PEGylated borotryptophan derivative has the following chemical formula (Ic):

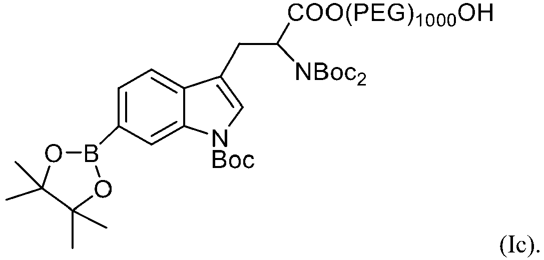

5. A PEGylated boronotryptophan derivative, having the following chemical formula (II): 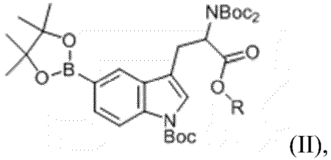 wherein R is polyethylene glycol (PEG).

6. The PEGylated boronotryptophan derivative of claim 5, wherein the PEG is PEG₂₀₀, and when the PEG is PEG₂₀₀, the PEGylated borotryptophan derivative has the following chemical formula (IIa):

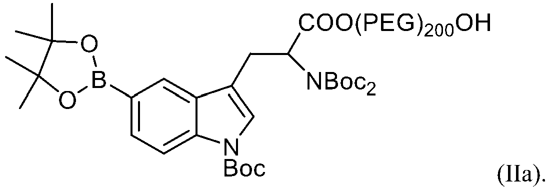

7. The PEGylated boronotryptophan derivative of claim 5, wherein the PEG is PEG₆₀₀, and when the PEG is PEG₆₀₀, the PEGylated borotryptophan derivative has the following chemical formula (IIb):

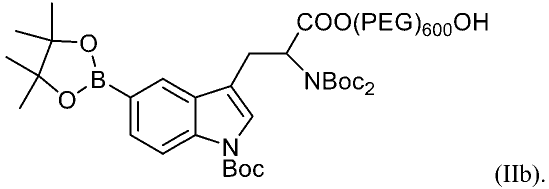

8. A PEGylated boronotryptophan derivative, having the following chemical formula (III): 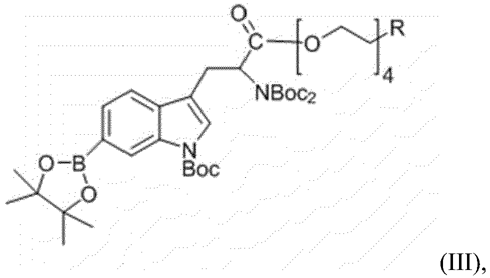 wherein R is OTs or F, and the PEGylated boronotryptophan derivative comprises PEG₂₀₀.

9. The PEGylated boronotryptophan derivative of claim 8, wherein when R is OTs, the PEGylated boronotryptophan derivative has the following chemical formula (IIIa):

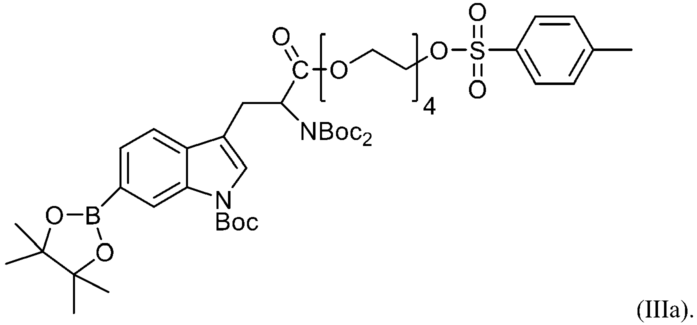

10. The PEGylated boronotryptophan derivative of claim 8, wherein when R is F, the PEGylated boronotryptophan derivative has the following chemical formula (IIIb):

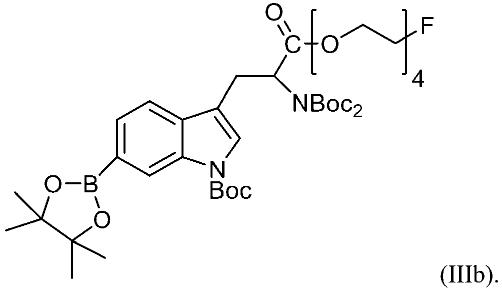

11. The PEGylated boronotryptophan derivative of claim 10, wherein the F is radioactive isotope of fluorine-18 (¹⁸F).

12. A method for preparing the PEGylated boronotryptophan derivative of any one of claims 1-4, comprising adding HBTU, DMAP and NEt₃ to the compound of chemical formula (I), wherein R of the compound of chemical formula (I) is H.

13. A method for preparing the PEGylated boronotryptophan derivative of any one of claims 5-7, comprising adding HBTU, DMAP and NEt₃ to the compound of chemical formula (II), wherein R of the compound of chemical formula (II) is H.

14. A pharmaceutical composition, comprising the PEGylated boronotryptophan derivative of any one of claims 1-11, and a pharmaceutically acceptable carrier.

15. The pharmaceutical composition of claim 14, wherein the pharmaceutical composition is in a dosage form for parenteral administration.

16. A use of the PEGylated boronotryptophan derivative of any one of claims 1-11 for the manufacture of a medicament for treating tumor.

17. A use of the PEGylated boronotryptophan derivative of any one of claims 1-11 for the manufacture of a formulation for positron emission tomography (PET) scanning for assessing pharmacokinetics for boron neutron capture therapy (BNCT) of tumor and quantifying drug concentrations to calculate pharmacokinetics.

18. The use of claim 16 or 17, wherein the tumor is a brain tumor.

19. The use of claim 18, wherein the brain tumor is glioma.

20. A method for assessing pharmacokinetics for boron neutron capture therapy of tumor, comprising using the PEGylated boronotryptophan derivative of any one of claims 1-11 for positron emission tomography (PET) scanning.
